# EUROPEAN PATENT SPECIFICATION

(11) **EP 2 398 511 B1**
(45) Date of publication and mention of the grant of the patent: **17.04.2013**
(21) Application number: 10713514.7
(22) Date of filing: 22.02.2010
(51) Int. Cl.: A61L 17/04, A61L 27/50, C08J 5/00, D01F 2/00, D01F 4/00, D01F 6/00, D01F 9/00, A61L 31/14, A61L 27/14, A61L 29/14

(54) **MEDICAL DEVICE AND METHOD FOR MAKING THE SAME**
MEDIZINISCHER ARTIKEL UND VERFAHREN ZU SEINER HERSTELLUNG
DISPOSITIF MÉDICAL ET SON PROCÉDÉ DE FABRICATION

(30) Priority: 21.02.2009 US 154384 P
(43) Date of publication of application: 28.12.2011
(73) Proprietor: Sofradim Production, 01600 Trévoux (FR); Codivien LP, New Haven CT 06511 (US)
(72) Inventor: HADBA, Ahmad, Robert, Middlefield CT 06455 (US); LADET, Sébastien, F-69006 Lyon (FR)
(74) Representative: Maureau, Philippe
(86) International application number: PCT/IB2010/000673
(87) International publication number: WO 2010/095057

(56) References cited:
- EP-A2- 2 014 308
- WO-A1-2008/031525
- WO-A2-2007/035296
- US-A1- 2007 178 133
- US-A1- 2008 314 289

## Description

### BACKGROUND

### Technical Field

The present disclosure relates to implantable devices having an activated surface.

### Background of Related Art

Methods for making monofilaments that are suitable to fabricate surgical articles, such as sutures, generally include the steps of extruding at least one bioabsorbable or nonbioabsorbable polymer to provide filaments, drawing or stretching the solidified filaments to achieve molecular orientation, and annealing the drawn filaments to relieve internal stresses.

Various spinning methods may be employed, such as melt spinning, gel spinning, wet or dry spinning, and reaction spinning. Melt spinning uses heat and potentially shear to melt the fiber-forming polymer to a viscosity suitable for extrusion through the die or spinneret. After exiting the die, the fiber solidifies by cooling in air or a suitable chilled fluid bath. In solvent spinning, the fiber-forming polymer is dissolved in a suitable organic solvents or solvent mixture to result in a fluid with suitable viscosity for extrusion through a spinneret. The difference between wet and dry spinning is the means by which the fiber solidifies. In dry spinning, the fiber solidifies as the solvent evaporates under a stream of air or inert gas. In wet spinning, the fiber forms by precipitating from solution as a result of dilution in a non-solvent bath or chemical reaction with a crosslinker in the solvent bath. Gel spinning refers to a process similar to solvent spinning except that the polymer is not fully dissolved in the solvent- a high polymer content is used in the process. The chains of the partially solvated polymer are aligned by the shear during the extrusion process. The filaments are further drawn as they are passed through a gas drying then a wet precipitating bath. The resulting fibers have an unusually high degree of allignmnet and high tensile strength relative to conventional melt or solvent spinning techniques. Reaction spinning involves the formation of filaments from reactive polymers or prepolymers and monomers that are further polymerized and cross-linked during the extrusion process or after the fiber or filament is formed.

Click chemistry refers to a collection of reactions capable of forming a highly reliable molecular connection in solution or bulk state. Click chemistry reactions may be highly selective, high yield reactions which should not interfere with one another as well as other reactions.

Document EP 2 014 308 A2 discloses a method for grafting a heparin to a biocompatible polyester via a click chemistry process. Document US2008/0314289 discloses a polyester amide (PEA) comprising free carboxylic acid pendant groups. Document W02008/031525 discloses the preparation of a hydrogel by click chemistry reactions. Document US2007/0178133 discloses a medical device comprising a base material in combination with a first curable functional group and a second curable functional group. Document W02007/035296 discloses a hydrogel composition comprising a hydrophilic polymer residue and a crosslinker residue.

It would be desirable to make filaments useful in making surgical devices by extruding a mixture containing first and second precursors functionalized for crosslinking by click chemistry and aided by the process controls of the spinning process, such as temperature, pressure, and time.

### SUMMARY

A first aspect of the invention is a method of forming a medical device comprising:
forming a desired shape from a polymer possessing a core and at least one functional group known to have click reactivity, whereby a medical device with an activated surface is produced.

In the present application, unless otherwise specified, the expressions "functional group", "functional group known to have click reactivity" and "reactive member" are used interchangeably to designate a functional group known to have click reactivity.

In the present application, unless otherwise specified, the expression "functionalized polymer" means the polymer possessing the functional group as defined herein.

Another aspect of the invention is a medical device comprising a polymer possessing a core and a functional group known to have click reactivity.

In embodiments, the core comprises synthetic materials selected from alpha-hydroxy acids (e.g. lactic acid, glycolic acid, and the like), lactide, glycolide, ∈-caprolactone, δ-valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, hydroxy alkanoates (e.g. γ-hydroxyvalerate, β-hydroxypropionate, 3-hydroxybuterate, and the like), poly (ortho esters), tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof.

In embodiments, the core comprises biodegradable polymers selected from collagen, cellulose, poly (amino acids), polysaccharides, hyaluronic acid, gut, copolymers and combinations thereof.

In embodiments, the core comprises non-degradable polymers selected from fluorinated polymers (e.g.fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, copolymers and combinations thereof.

In embodiments, the functional group known to have click reactivity is selected from the group consisting in amine, sulfate, thiols, hydroxyl, azides, alkynes, alkenes, carboxyl groups, aldehyde groups, sulfone groups, vinylsulfone groups, isocyanate groups, acid anhydride groups, epoxide groups, aziridine groups, episulfide groups, groups such as -CO₂N(COCH₂)₂, CO₂N(COCH₂)₂, -CO₂H, -CHO, -CHOCH₂, -N=C=O, -SO₂CH=CH₂ -N(COCH)₂, -S-S-(C₅H₄N), and/or groups of the following structures wherein X is halogen and R is hydrogen or C₁ to C₄ alkyl:

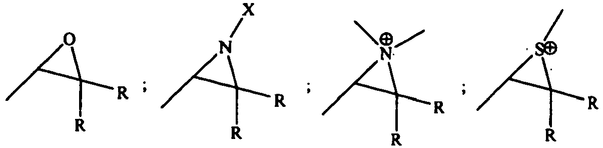

For example, the functional group known to have click reactivity is selected from the group consisting in thiols, azides, alkynes and alkenes. The functional group known to have click reactivity may be a thiol. The functional group known to have click reactivity may be an azide.

The functional group known to have click reactivity may be an alkyne.

The functional group known to have click reactivity may be an alkene.

In embodiments, the shape or the medical device is selected from a fiber, sheet, rod, staple, clip, needle, tube, foam and combinations thereof.

In embodiments, the polymer is melt extruded to form a fiber.

In embodiments, the polymer being chitosan, the polymer is formed into a fiber by spinning an anisotropic solution of chitosan. By "chitosan" is meant herein chitosan or a derivative of chitin or of chitosan.

In embodiments, the core being collagen, the polymer is formed into a fiber by gel spinning. By "collagen" is meant herein collagen or collagen derivatives.

In embodiments, the medical device is a fiber.

Implantable medical devices with an activated surface in accordance with this disclosure are fabricated from a polymer possessing a core and at least one functional group known to have click reactivity. In embodiments, the implantable medical device includes is molded, extruded or cast from such a polymer and has a plurality of functional groups known to have click reactivity at the surface thereof.

Another aspect of the invention is a method comprising:
forming a desired shape from a polymer possessing a core and at least one functional group known to have click reactivity, whereby a medical device with an activated surface is produced.

Another aspect of the invention is a medical device prepared from a polymer possessing a functional group having click reactivity.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure.
Fig. 1 is a schematic illustration of an apparatus which is suitable for carrying out a fiber manufacturing process in accordance with the present disclosure.
Figs. 2 and 3 schematically illustrate an apparatus which is suitable for carrying out an alternate fiber manufacturing process in accordance with the present disclosure.
Fig. 4 schematically illustrates an apparatus which is suitable for carrying out yet another fiber manufacturing process in accordance with the present disclosure.

### DETAILED DESCRIPTION OF EMBODIMENTS

Implantable medical devices in accordance with the present disclosure are prepared from a polymer having at least one functional group known to have click reactivity. Once fabricated into a desired shape, the implantable medical device will have a plurality of functional groups known to have click reactivity at the surface thereof.

The polymer used to make implantable medical devices in accordance with the present disclosure possess a core that is functionalized with one or more reactive members.

The core of the polymer may be any suitable biocompatible polymer. The core may be a homopolymer or a copolymer, including random copolymer, block copolymer, or graft copolymer. The core may be a linear polymer, a branched polymer, or a dendrimer. The core of may be a natural material or a synthetic material and may be bioabsorbable or non-bioabsorbable. It should of course be understood that any combination of natural, synthetic, bioabsorbable and non-bioabsorbable materials may be used to form the implantable medical device.

Some non-limiting examples of synthetic materials from which the core may be prepared include, but are not limited to polymers such as those made from alpha-hydroxy acids (e.g. lactic acid, glycolic acid, and the like), lactide, glycolide, ∈-caprolactone, δ-valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, hydroxy alkanoates (e.g. γ-hydroxyvalerate, β-hydroxypropionate, 3-hydroxybuterate, and the like), poly (ortho esters), tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof. Suitable natural biodegradable polymers include collagen, cellulose, poly (amino acids), polysaccharides, hyaluronic acid, gut, copolymers and combinations thereof.

Examples of suitable non-degradable polymers from which the substrate of the medical devices described herein may be made include, but are not limited to fluorinated polymers (e.g.fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, copolymers and combinations thereof.

In preparing implantable medical devices in accordance with the present disclosure, the polymer may be commercially available pre-functionalized cores or may be synthesized. It is contemplated that a plurality of different reactive members may be present and that they may be terminally located, or alternatively located along the length of the polymer chain. In embodiments, the polymer has from about 2 to about 50 reactive members.

Click chemistry refers to a collection of reactive members having a high chemical potential energy capable of producing highly selective, high yield reactions. The reactive members react to form extremely reliable molecular connections in most solvents, including physiologic fluids, and often do not interfere with other reagents and reactions. Examples of click chemistry reactions include Huisgen cycloaddition, Diels-Alder reactions, thiol-alkene reactions, and maleimide-thiol reactions.

Huisgen cycloaddition is the reaction of a dipolarophile with a 1,3-dipolar compound that leads to 5-membered (hetero)cycles. Examples of dipolarophiles are alkenes and alkynes and molecules that possess related heteroatom functional groups (such as carbonyls and nitriles). 1,3-Dipolar compounds contain one or more heteroatoms and can be described as having at least one mesomeric structure that represents a charged dipole. They include nitril oxides, azides, and diazoalkanes. Metal catalyzed click chemistry is an extremely efficient variant of the Huisgen 1,3-dipolar cycloaddition reaction between alkyl-aryly-sulfonyl azides, C-N triple bonds and C-C triple bonds which is well-suited herein. The results of these reactions are 1,2 oxazoles, 1,2,3 triazoles or tetrazoles. For example, 1,2,3 triazoles are formed by a copper catalyzed Huisgen reaction between alkynes and alkly/aryl azides. Metal catalyzed Huisgen reactions proceed at ambient temperature, are not sensitive to solvents, i.e., nonpolar, polar, semipolar, and are highly tolerant of functional groups. Non-metal Huisgen reactions (also referred to as strain promoted cycloaddition) involving use of a substituted cyclooctyne, which possesses ring strain and electron-withdrawing substituents such as fluorine, that together promote a [3+ 2] dipolar cycloaddition with azides are especially well-suited for use herein due to low toxicity as compared to the metal catalyzed reactions. Examples include DIFO and DIMAC. Reaction of the alkynes and azides is very specific and essentially inert against the chemical environment of biological tissues. One reaction scheme may be represented as: 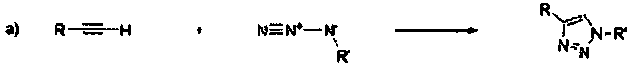 where R and R' are the core of the polymer.

The Diels-Alder reaction combines a diene (a molecule with two alternating double bonds) and a dienophile (an alkene) to make rings and bicyclic compounds. Examples include:

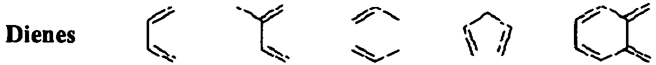

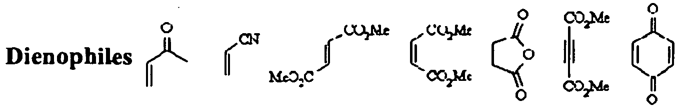

The thiol-alkene (thiol-ene) reaction is a hydrothiolation, i.e., addition of RS-H across a C=C bond. The thiol-ene reaction proceeds via a free-radical chain mechanism. Initiation occurs by radical formation upon UV excitation of a photoinitiator or the thiol itself. Thiol-ene systems form ground state charge transfer complexes and therefore photopolymerize even in the absence of initiators in reasonable polymerization times. However, the addition of UV light increases the speed at which the reaction proceeds. The wavelength of the light can be modulated as needed, depending upon the size and nature of the constituents attached to the thiol or alkene. A general thiol-ene coupling reaction mechanism is represented below: 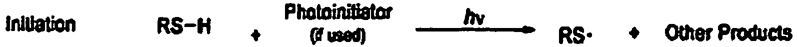 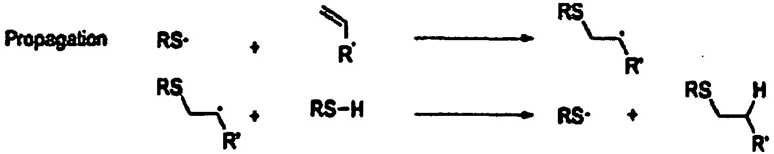 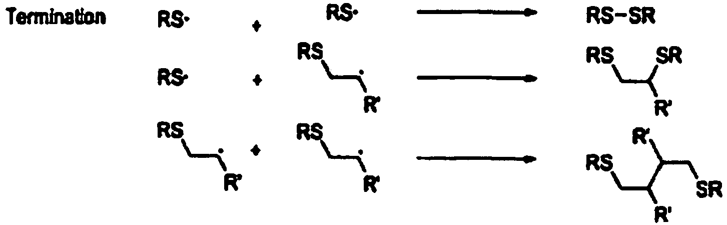 Thus, suitable reactive members that may be applied to the core include, for example, an amine, sulfate, thiol, hydroxyl, azide, alkyne, alkene, carboxyl groups, aldehyde groups, sulfone groups, vinylsulfone groups, isocyanate groups, acid anhydride groups, epoxide groups, aziridine groups, episulfide groups, groups such as -CO₂N(COCH₂)₂, -CO₂N(COCH₂)₂, -CO₂H, -CHO, -CHOCH₂, -N=C=O, -SO₂CH=CH₂ -N(COCH)₂, -S-S-(C₅H₄N), and/or groups of the following structures wherein X is halogen and R is hydrogen or C₁ to C₄ alkyl: 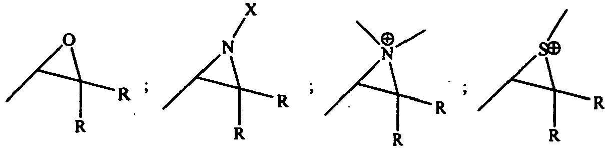 In particular, the functional group known to have click reactivity is selected from the group consisting in thiols, azides, alkynes and alkenes.
The core of the polymer can be provided with click reactive members using any variety of suitable chemical processes.

For example, the monomers from which the core is made can be functionalized so that the reactive members appear along the length of the core. In such embodiments, monomers can be initially functionalized with a group such as a halogen to provide a reactive site at which the desired first click reactive member can be attached after polymerization. Thus, for example, a cyclic lactone (e.g., glycolide, lactide, caprolactone, etc.) can be halogenated and then polymerized using known techniques for ring opening polymerization. Once polymerized, the halogenated sites along the resulting polyester chain can be functionalized with the first reactive member. For example, the halogenated polyester can be reacted with sodium azide to provide azide groups along the polymer chain or with propagyl alcohol to provide alkyne groups along the polymer chain. See, R. Riva et al., Polymer 49, pages 2023-2028 (2008) for a description of such reaction schemes. In another example, a propargyl group may be introduce into a cyclic carbonate monomer to form 5-methyl-5-propargyloxycarbonyl-1,3-dioxan-2-one (MPC) which is polymerizable with lactide to form p(LA-co-MPC). See, Q. Shi et al., Biomaterials, 29, pages 1118-1126 (2008). Alternatively, the polymer or copolymer backbone may be halogenated using methods similar to those described by Nottelet et al., Biomaterials, 27, pages 4948-4954 (2006). Once halogenated, the backbone can be functionalized with a click reactive functionality by reacting it with a hydroxyacid under condition described by Shi et al. Biomaterials, 29, pages 1118-1126 (2008) followed by reaction with sodium azide. The halogen may also be converted directly to the alkyne by reacting it with an alcoholic alkyne such as propargyl alcohol.

Those skilled in the art reading this disclosure will readily envision chemical reactions for activating other core materials to render them suitable for use as precursors in the presently described methods.

The polymers may be fabricated into any desired physical form. The polymeric substrate may be fabricated for example, by spinning, casting, molding or any other fabrication technique known to those skilled in the art. The polymer may be made into any shape, such as, for example, a fiber, sheet, rod, staple, clip, needle, tube, foam, or any other configuration suitable for a medical device. Where the polymer is in the form of a fiber, the fiber may be formed into a textile using any known technique including, but not limited to, knitting, weaving, tatting and the like. It is further contemplated that the polymer may be a non-woven fibrous structure.

The polymer can be part of any medical device of being implanted at a target location. Some non-limiting examples include monofilaments, multifilaments, surgical meshes, ligatures, sutures, staples, patches, slings, foams, pellicles, films, barriers, stents, catheters, shunts, grafts, coil, inflatable balloon, and the like. The implantable device can be intended for permanent or temporary implantation.

In fabricating the medical device, the polymer may take the form of any solution, suspension, semi-solid, or solid material capable of allowing the polymer to be fabricated into a desired shape. The polymer may be in granular, pellet, or powder form, or alternatively, may be in a dilute solution. Suitable solvents which may be utilized to form a dilute solution include any biocompatible solvent within the purview of those skilled in the art which will not interfere with the reaction of the reactive members of the first and second precursors. Suitable solvents which may be utilized include, for example, polar solvents such as water, ethanol, triethylene glycol, dimethyl sulfoxide, glymes (such as diglyme, triglyme, tetraglyme, and the like), polyethylene glycols, methoxy-polyethylene glycols, dimethylformamide, dimethylacetamide, gamma-butyrolactone, n-methylpyrollidone, ketones such as methyl ethyl ketone, cyclohexanone, diethylene glycol momethyl ether acetate, diethylene glycol monobutyl ether acetate, diethylene glycol monomethyl ether, diethylene glycol monoethyl ether, diethylene glycol monobutyl ether, diethylene glycol monoisobutyl either, diisobutyl ketone, diacetone alcohol, ethyl amyl ketone, ethyl lactate, and the like. In other embodiments, solvents such as tetrahydrofuran, ethyl acetate, isopropyl acetate, butyl acetate, isopropanol, butanol, acetone, and the like, may be utilized. In embodiments, combinations of any of the foregoing solvents may be utilized to form a dilute solution. The amount of solvent used will depend on a number of factors, including the particular first precursor, second precursor, or combination thereof that are to be employed and the intended end use of the composition.

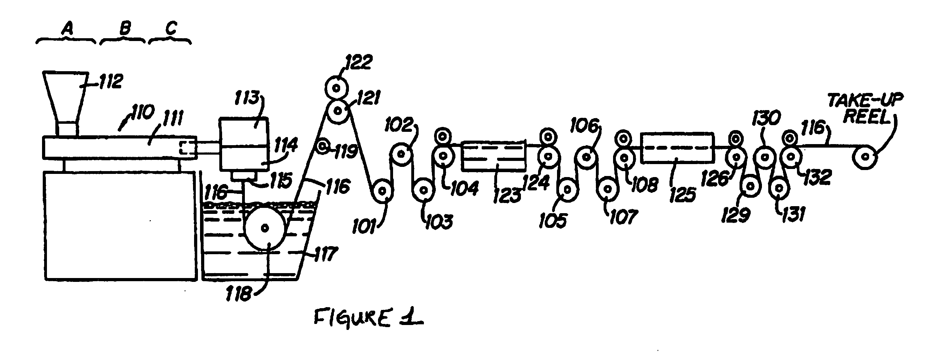

In embodiments, the polymer is melt extruded to form a fiber. Known spinning apparatuses can be used for the production of filaments, in accordance with the present disclosure. FIG. 1 schematically illustrates a filament manufacturing operation in accordance with the disclosure. Extruder unit 110 is of a known or conventional types and is equipped with controls for regulating the temperature of barrel 111 in various zones thereof, e.g., progressively higher temperatures in three consecutive zones, A, B, and C along the length of the barrel. The first and second precursors to be spun into filaments are introduced to the extruder through hopper 112. Prior to or during placement in hopper 112, the first precursor is combined with the second precursor and mixed in a one-pot process. Adding heat during the mixing and/or extruding steps aids in the curing time of the first and second precursors, as faster curing rates are observed at higher temperatures.

Motor-driven metering pump 113 delivers the melt extruded first and second precursor mixture at a constant rate and with high pressure to spin pack 114 and thereafter through spinneret 115 possessing one or more orifices of desired diameter to provide a molten monofilament 116 which then enters quench bath 117, e.g., containing water, where the monofilament solidifies. The distance monofilament 116 travels after emerging from spinneret 115 to the point where it enters quench bath 117, i.e., the air gap, can vary. If desired, a chimney (not shown), or shield, can be provided to isolate monofilament 116 from contact with air currents which might otherwise affect the cooling of the monofilament in an unpredictable manner. In general, barrel zone A of the extruder can be maintained at a temperature of from about 100°C to 220°C, zone B at from about 160°C to 230°C and zone C at from about 170°C to about 240°C. Additional temperature parameters include: metering pump block 113 at from about 170°C to about 230°C, spin pack 114 at from about 170°C to about 230°C, spinneret 115 at from about 170°C to about 230°C and quench bath at from about 10°C to about 80°C.

Monofilament 116 is passed through quench bath 117 around driven roller 118 and over idle roller 119. Optionally, a wiper (not shown) may remove excess water from the monofilament as it is removed from quench bath 117. On exiting the quench bath the monofilament is wrapped around a first godet 121 provided with nip roll 122 to prevent slippage which might otherwise result from the subsequent stretching operation; and subsequently wrapped around godets 101, 102, 103 and 104 or any other suitable godet arrangement. Monofilament 116 passing from godet 104 is stretched, e.g., with stretch ratios on the order of from about 3:1 to about 10:1 and preferably from about 4:1 to about 7:1, to effect its orientation and thereby increase its tensile strength.

In the stretching operation, monofilament 116 may be drawn through hot water (or other suitable liquid medium) draw bath 123 by means of godets 124, 105, 106, 107 and 108 or any other suitable arrangement of godets which rotate at a higher speed than godet 104 to provide the desired stretch ratio. The temperature of hot water draw bath 123 is advantageously from about 30°C to about 90°C and preferably is from about 30°C to about 50°C. In an alternative stretching operation, generally preferred for smaller sutures sizes, e.g., sizes 3/0 to 8/0, monofilament 116 may be drawn by godets 124, 105, 106, 107, and 108 or any other suitable godet arrangement through hot air convection oven chamber 123 at a temperature of from about 30°C to about 140°C, and preferably from about 50°C to about 130°C to provide the desired amount of stretch.

Following the stretching operation, monofilament 116 optionally may be subjected to an on-line annealing and/or additional stretching without shrinkage or relaxation with shrinkage operation as a result of which the monofilament shrinks. In the process of FIG. 1, on-line annealing with or without relaxation when desired is accomplished by driving monofilament 116 by godets 126, 129, 130, 131, and 132 or any other suitable godet arrangement through second hot air oven chamber 125 at a temperature of from about 40°C to about 150°C, and preferably from about 60°C to about 130°C. During the relaxation process, at these temperatures, monofilament 116 will generally recover to within about 80 to about 97 percent, and preferably to within about 95 percent, of its pre-annealed length to provide the finished suture. For relaxation, the third godet rotates at a slower speed than the second godet thus relieving tension on the filament.

Annealing of the suture also may be accomplished without shrinkage of the suture. In carrying out the annealing operation, the desired length of suture may be wound around a creel and the creel placed in a heating cabinet maintained at the desired temperature, e.g. about 60°C to about 130°C. After a suitable period of residency in the heating cabinet, e.g., about 18 hours or so, the suture will have undergone essentially no shrinkage. Variables such as the annealing temperatures, time, and pressure may affect the curing time of the fibers as well. The creel may be rotated within the heating cabinet in order to insure uniform heating of the monofilament or the cabinet may be of the circulating hot air type in which case uniform heating of the monofilament will be achieved without the need to rotate the creel. Thereafter, the creel with its annealed suture is removed from the heating cabinet and when returned to room temperature, the suture is removed from the creel, conveniently by cutting the wound monofilament at opposite ends of the creel. The annealed sutures are then ready to be packaged and sterilized.

In embodiments, surface activated fibers are formed from functionalized polymers having chitin or chitin derivative cores that have been functionalized with click reactive members. Such polymers can be formed into fibers according to the present disclosure by spinning from anisotropic solution. Suitable methods for solution spinning chitin or chitin derivative fibers in general are disclosed in European Patent Nos. EP0328050A2 and EP0077098A2, the entire disclosures of which are incorporated herein by this reference. Such fibers can have tensile properties which typically fall between 4-8 g/d tenacity and 150-250 g/d initial modulus.

High strength chitosan fibers can be prepared by spinning an aniostropic solution of chitosan or a derivative of chitin or chitosan through an inert gas and into a coagulating bath, removing the as-spun fiber and treating it with alkali to remove N-acetyl, O-acetyl or other pendant groups at the 2, 3 and 6 carbon positions of the glucosamine repeating unit. Treatment of fibers is by immersion of the fibers into a solution of NaOH. With fine denier fibers, e.g., 4-5 dpf., a 5 minute immersion at 70° C. in a 50% wt. solution of NaOH is satisfactory. A 2-3 hr. exposure at 80° C. in a 30% wt. solution is useful with chitosan acetate formate fiber. With chitosan acetate, temperatures in the range of 80° to 116° C. at NaOH concentration of 30% have been found useful with the higher temperatures requiring less time for completion of the reaction. Severe treatments are generally to be avoided since they may cause excessive interfilament fusion and a product of inferior quality. Conversion of the starting fiber to a chitosan fiber is confirmed if the chitosan fiber is readily soluble in dilute (3-20% wt.) acetic acid.

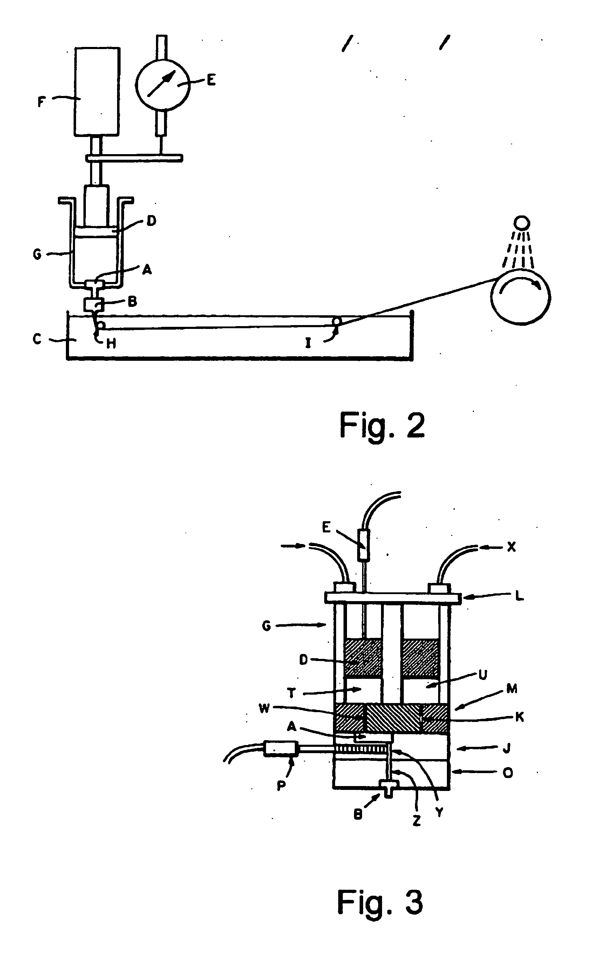

In using the apparatus of FIG. 2 an anisotropic solution of chitin or a chitin derivative is placed in spin cell (G). A piston (D) activated by hydraulic press (F) and associated with piston travel indicator (E) is positioned over the surface of the solution, excess air is expelled from the top of the cell and the cell is sealed. The spin cell is fitted at the bottom with the following screens (A) for solution filtration: four to six 325-mesh screens. The filtered solution is then passed into a spinneret pack (B) containing two or three 325-mesh screens. Solutions are extruded through an air gap at a controlled rate into a static bath (C) using a metering pump to supply pressure at piston (D). The fiber is passed around a pin (H), pulled through the bath, passed under a second pin (I) and wound onto a bobbin. The air gap between the spinneret face and the coagulation bath is typically 0.6 to 2.0 cm. The coagulation bath temperature is generally held below 100° C.

In using the apparatus of FIG. 3, filter plate (J) is replaced by mixing plate (R). Polymer dope is placed in cylinder bore (T) and then piston (D) and cap plate (L) is fitted to the spin cell (G). A driver fluid (e.g. water) is pumped into the upper part of bore (T) through feed line (F). The piston (D) is displaced by the driver fluid, thereby pushing the polymer dope through passages (W), (S) in mixing plate (R) and then through passage (K) in distribution plate (M) into second cylinder bore (U). This process is then reversed by pumping fluid through feed line (X). The aforementioned forward and reverse process is repeated several times to effect a mixing of the polymer dope. Component (E) acts to sense the position of cylinder (D).

After mixing is complete (about 30 cycles), mixing plate (R) is replaced by filter plate (J) and polymer dope is extruded from bore (T) through passage (W), through filter pack (A) containing 2 Dutch Twill Weave 165×800 mesh screens, through passage (Y) in filter plate (J) and passage (Z) in spinneret mounting plate (O) and out of spin cell (G) through spinneret (B). The extruded dope is spun into a bath and taken up as described for FIG. 2. Pressure of the polymer dope during spinning is measured by pressure transducer (P).

In other embodiments, surface activated fibers are formed from functionalized polymers having collagen or collagen derivative cores. Such polymers can be formed into fibers according to the present disclosure by gel spinning. Suitable methods for gel spinning collagen fibers in general are disclosed in U.S. Patent Nos. 5,562,946 and 5,911,942, the entire disclosures of which are incorporated herein by this reference.

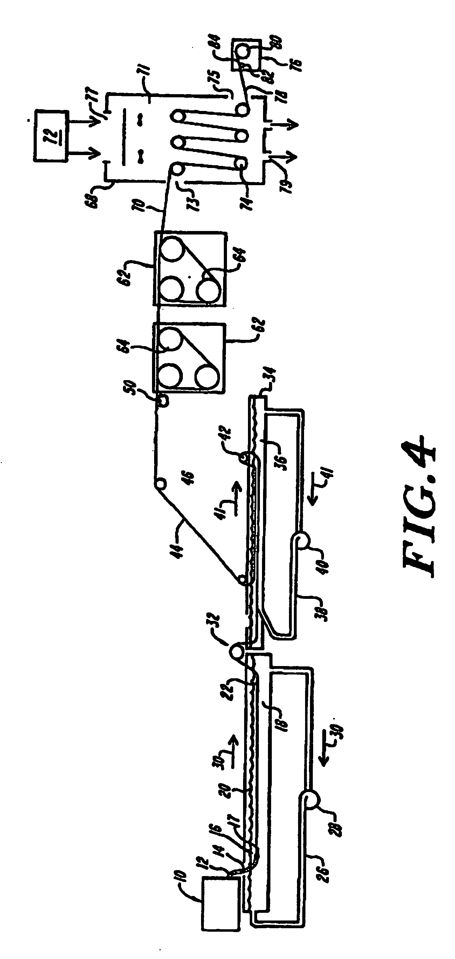

In an illustrative apparatus for gel spinning such fibers shown in Fig. 4, collagen reservoir chamber 10 holds a liquid collagen solution. In one embodiment, a suitable chamber is a stainless steel syringe. Reservoir tube 12 is attached to collagen reservoir chamber 10 for directing collagen solution from collagen reservoir chamber 10 through infusion pump 14 to spinneret 16. Infusion pump 14 is capable of raising the pressure of the collagen material such that it can be extruded through spinneret nozzle 17 of spinneret 16. In embodiments, a positive displacement metering pump is used. Spinneret 16 can be single bore or multiple bore to produce monofilament or multifilament fibers respectively. The spinneret bores can be of various diameters or have tapered profiles to form fibers of different sizes and tensile strengths. Co-component fibers can be produced with other specialized spinnerets as are known in the art. In one embodiment, spinneret nozzle 17 has diameters in the range of between about 100 and 1,000 microns.

Coagulation bath 18 has a coagulation solution 20 that can cause the liquid collagen to form a collagen gel, such as a 0.75% alkaline alginic acid in a boric acid buffer or sugar solutions or polyethylene glycol solution which also has hydrophilic properties. The opening of spinneret is immersed in a flowing coagulation solution 20. Coagulation bath 18 is suitably sized for allowing extrusion of fiber from spinneret 16 through coagulation solution 20 while having a sufficient residency time for collagen gel fiber 22 to form. Coagulation bath 18 can be heated and instrumented for monitoring the relevant process variables, such as temperature, pH and velocity. Coagulation bath 18 allows collagen gel fiber 22 to be formed in a horizontal trough or in a tube or vertically in a tube. Coagulation bath 18 is configured to allow circulation of coagulation solution 20 through recirculating loop 26 by circulating pump 28. Coagulation bath flow can be in the same direction 30 of fiber travel. At the end of the coagulation bath 18, roller 32 is for directing fiber out of the coagulation bath. Roller 32 is motorized and can be activated to wind collagen gel fiber 22 and subsequently tow collagen gel fiber 22 at desired speeds.

Dehydrating bath 34 is adjacent to roller 32 and coagulation bath 18 and is configured to allow fiber 22 to be drawn into dehydrating bath 34 from roller 32. Dehydrating bath 34 holds dehydrating solution 36, such as 90% ethanol, which allows further dehydration and annealing of the fiber and promotes polymerization of the collagen to improve fiber strength. An example of another suitable dehydration solution composition is acetone. Dehydrating bath 34 is configured to allow variable circulation of dehydrating solution 36 through recirculating loop 38 by circulating pump 40 which can be adjusted directionally, such as direction 41 or in the opposite direction. Return rollers 42, which can be near each end of dehydrating bath 34, allow the fiber path to be lengthened by doubling back to make any number of multiple passes through dehydrating bath 34 to allow further dehydration and promote polymerization of the collagen.

Partially dehydrated fiber 44 is wound around roller 46 to second roller 50 and then to stretching roller means 62, wherein the fiber can undergo a controlled deformation by being stretched between two groups of rollers 64 rotating at slightly different rates of speed. The speed of rotation of rollers 64 can be precisely controlled with digital microprocessors arranged in a closed feedback loop. The fibers are wrapped around each roller 64 several times to prevent fiber slippage relative to the roller surfaces. Roller 64 surfaces can be made of a polymer or a hardened metal resistant to corrosion. Roller 64 rotations can be adjusted individually to allow the fiber to be stretched beyond the elastic yield point to produce a longer fiber of reduced diameter. Stretching roller means 62 can operate under semi-dry or dry conditions and also under high moisture content atmosphere.

Drying cabinet 68 has opening 73 for receiving stretched fiber 70 from stretching rollers 62. Drying cabinet 68 has passage 71 through drying cabinet 68 for receiving warm, dry filtered air or a dry inert gas, such as dry nitrogen gas, from gas source 72 at a suitable temperature and humidity for drying stretched fiber 70. The air can be passed through air passage opening 77 into passage 71 and exiting from air passage opening 79. In embodiments, the temperature of the air is between about 35° C. and 39° C. The humidity is in the range of between 10 and 20 percent relative humidity. Drying cabinet 68 has a series of rollers 74 which allows stretched fiber 70 to remain in drying cabinet 68 while being rolled, thereby increasing the residence time of fiber 70 in drying cabinet 68. Drying cabinet rollers 74 are adjustable in distance between each other and to compensate for the fiber line speed. Drying cabinet rollers 74 can be driven at a surface roller speed that can be synchronized with that of stretching roller means 62. Drying cabinet 68 has a door to provide access to the rollers for threading the leader thread.

Take-up winder 76 is for receiving dried fiber 78 from exit 75 of drying cabinet 68. Take-up winder 76 has spool 80 for receiving dried fiber on a removable spindle bobbin. Take-up winder 76 has a slip clutch 82 to provide a constant fiber line tension and fiber line speed as the spooled fiber rotates radially around spool 80. Fiber spool 80 can wind the fiber level or by randomly winding with the take-up winder 76.

In each case (melt extrusion, solution spinning or gel spinning), the resulting fiber possesses click reactive functional groups at the surface thereof.

The present medical devices may further be use for delivery of a bioactive agent. Thus, in some embodiments, at least one bioactive agent may be combined with polymer prior to forming the medical device and/or may be separately applied to the formed device.. The agents may be freely admixed with the functionalized polymer or may be tethered to the polymers through any variety of chemical bonds. In these embodiments, the present devices can also serve as a vehicle for delivery of the bioactive agent. The term "bioactive agent," as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present devices in any suitable form of matter, e.g., films, powders, liquids, gels and the like.

Examples of classes of bioactive agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors and enzymes. It is also intended that combinations of bioactive agents may be used.

Anti-adhesive agents can be used to prevent adhesions from forming between the implantable medical device and the surrounding tissues opposite the target tissue. In addition, anti-adhesive agents may be used to prevent adhesions from forming between the coated implantable medical device and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents which may be included as a bioactive agent in the bioactive coating of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the present disclosure.

Other bioactive agents which may be included as a bioactive agent in the coating composition applied in accordance with the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents which may be included in the coating composition include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; and ribozymes.

Devices formed in accordance with the present disclosure may be at least partially coated with a bioresorbable coating by a surface treatment for enhanced properties. For example, the coating may be collagen, chitosan, polysaccharides, or mixtures thereof. The polysaccharides may be hyaluronic acid, alginic acid, polyglucuronic acid, chitosan, starch, soluble cellulose derivatives, and mixtures thereof.

Medical devices having an activated surface in accordance with the present disclosure can be used for a variety of purposes. For example, in embodiments they may be used for drug delivery. In such embodiments, the drug to be delivered is functionalized with one or more reactive member that are complementary to the reactive members at the surface of the device. By "complementary" it is meant that the reactive members on the drug to be delivered are able to interact with the reactive members at the surface of the device to covalently bond the drug to be delivered to the surface of the device.

In other embodiments, the medical device having an activated surface in accordance with the present disclosure can be attached to biological tissue by functionalizing tissue with one or more reactive member that are complementary to the reactive members at the surface of the device. Biological tissue can be provided with reactive member that are complementary to the reactive members at the surface of the device by conjugation of such groups to various components of tissue such as proteins, lipids, oligosaccharides, oligonucleotides, glycans, including glycosaminoglycans. In embodiments, the complementary groups are attached directly to components of the tissue. In other embodiments, the complementary groups are attached to components of the tissue via a linker. In either case, situating the complementary groups on the tissue can be accomplished by suspending the reactive member in a solution or suspension and applying the solution or suspension to the tissue such that the reactive member binds to a target. The solution or suspension may be poured, sprayed or painted onto the tissue, whereupon the reactive members are incorporated into the tissue.

Those skilled in the art reading this disclosure will readily envision other uses for the activated medical devices described herein.

While several embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of embodiments. Those skilled in the art will envision other modifications within the scope and spirit of the claims appended hereto.

## Claims

1. A method of forming a medical device comprising:
forming a desired shape from a polymer possessing a core and at least one functional group known to have click reactivity, whereby a medical device with an activated surface is produced, wherein the medical device is a fiber and wherein the functional group known to have click reactivity is selected from the group consisting in thiols, azides, alkynes and alkenes.

2. The method of claim 1, wherein the core comprises synthetic materials selected from alpha-hydroxy acids (e.g. lactic acid, glycolic acid, and the like), lactide, glycolide, ε-caprolactone, δ-valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, hydroxy alkanoates (e.g. γ-hydroxyvalerate, β-hydroxypropionate, 3-hydroxybuterate, and the like), poly (ortho esters), tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof.

3. The method of claim 1, wherein the core comprises biodegradable polymers selected from collagen, cellulose, poly (amino acids), polysaccharides, hyaluronic acid, gut, copolymers and combinations thereof.

4. The method of claim 1, wherein the core comprises non-degradable polymers selected from fluorinated polymers (e.g.fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, copolymers and combinations thereof.

5. The method of any one of claims I to 4, wherein the polymer is melt extruded to form a fiber.

6. The method of claim 1, wherein the polymer being chitosan, the polymer is formed into a fiber by spinning an anisotropic solution of chitosan.

7. The method of claim 1, wherein the polymer being collagen, the polymer is formed into a fiber by gel spinning.

8. A medical device comprising a polymer possessing a core and a functional group known to have click reactivity, wherein the medical device is a fiber and wherein the functional group known to have click reactivity is selected from the group consisting in thiols, azides, alkynes and alkenes.

9. The medical device of claim 8, wherein the core comprises synthetic materials selected from alpha-hydroxy acids (e.g. lactic acid, glycolic acid, and the like), lactide, glycolide, ε-caprolactone, δ-valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, hydroxy alkanoates (e.g. γ-hydroxyvalerate, β-hydroxypropionate, 3-hydroxybuterate, and the like), poly (ortho esters), tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof.

10. The medical device of claim 8, wherein the core comprises biodegradable polymers selected from collagen, cellulose, poly (amino acids), polysaccharides, hyaluronic acid, gut, copolymers and combinations thereof.

11. The medical device of claim 8, wherein the core comprises non-degradable polymers selected from fluorinated polymers (e.g.fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, copolymers and combinations thereof.

12. The medical device of claim 8, wherein the functional group known to have click reactivity is a thiol.

13. The medical device claim 8, wherein the functional group known to have click reactivity is an azide.

14. The medical device of claim 8, wherein the functional group known to have click reactivity is an alkyne.

15. The medical device of claim 8, wherein the functional group known to have click reactivity is an alkene.

## Patentansprüche

1. Verfahren zum Bilden einer medizinischen Vorrichtung, umfassend:
Bilden einer gewünschten Form aus einem Polymer, das einen Kern und zumindest eine funktionelle Gruppe aufweist, von der bekannt ist, dass sie Click-Reaktivität besitzt, wodurch eine medizinische Vorrichtung mit einer aktivierten Oberfläche produziert wird, wobei die medizinische Vorrichtung eine Faser ist, und wobei die funktionelle Gruppe, von der bekannt ist, dass sie Click-Reaktivität besitzt, aus der Gruppe, bestehend aus Thiolen, Aziden, Alkynen und Alkenen, ausgewählt ist.

2. Verfahren nach Anspruch 1, wobei der Kern synthetische Materialien umfasst, ausgewählt aus alpha-Hydroxysäuren (z. B. Milchsäure, Glykolsäure und dergleichen), Lactid, Glycolid, ε-Caprolacton, δ-Valerolacton, Carbonaten (z. B. Trimethylencarbonat, Tetramethylencarbonat und dergleichen), Dioxanonen (z. B. 1,4-Dioxanon), 1,Dioxepanonen (z. B. 1,4-Dioxepan-2-on und 1,5-Dioxepan-2-on), Ethylenglykol, Ethylenoxid, Esteramiden, Hydroxyalkanoaten (z. B. γ-Hydroxyvalerat, β-Hydroxypropionat, 3-Hydroxybuterat und dergleichen), Poly(orthoestern), Tyrosincarbonaten, Polyimidcarbonaten, Polyiminocarbonaten wie Poly(bisphenol A-iminocarbonat) und Poly(hydrochinon-iminocarbonat), Polyurethanen, Polyanhydriden, Polymerarzneimitteln (z. B. Polydiflunisol, Polyaspirin und Proteintherapeutika) und Copolymeren und Kombinationen davon.

3. Verfahren nach Anspruch 1, wobei der Kern biologisch abbaubare Polymere umfasst, ausgewählt aus Kollagen, Cellulose, Poly(aminosäuren), Polysacchariden, Hyaluronsäure, Eingeweiden ("gut"), Copolymeren und Kombinationen davon.

4. Verfahren nach Anspruch 1, wobei der Kern nicht-abbaubare Polymere umfasst, ausgewählt aus fluorierten Polymeren (z. B. Fluorethylenen, Propylenen, fluorPEGs), Polyolefinen wie Polyethylen, Polyestern wie Polyethylenterephthalat (PET), Nylons, Polyamiden, Polyurethanen, Siliconen, Polyethylen mit äußerst hohem Molekulargewicht (UHMWPE), Polybutestern, Polyaryletherketon, Copolymeren und Kombinationen davon.

5. Verfahren nach einem der Ansprüche 1 bis 4, wobei das Polymer schmelzextrudiert wird, um eine Faser zu bilden.

6. Verfahren nach Anspruch 1, wobei das Polymer Chitosan ist, wobei das Polymer durch Spinning einer anisotropen Chitosanlösung in eine Faser geformt wird.

7. Verfahren nach Anspruch 1, wobei das Polymer Kollagen ist, wobei das Polymer durch Gelspinning in eine Faser geformt wird.

8. Medizinische Vorrichtung, umfassend ein Polymer mit einem Kern und einer funktionellen Gruppe, von der bekannt ist, dass sie Click-Reaktivität besitzt, wobei die medizinische Vorrichtung eine Faser ist, und wobei die funktionelle Gruppe, von der bekannt ist, dass sie Click-Reaktivität besitzt, aus der Gruppe, bestehend aus Thiolen, Aziden, Alkynen und Alkenen, ausgewählt ist.

9. Medizinische Vorrichtung nach Anspruch 8, wobei der Kern synthetische Materialien umfasst, ausgewählt aus alpha-Hydroxysäuren (z. B. Milchsäure, Glykolsäure und dergleichen), Lactid, Glycolid, ε-Caprolacton, δ-Valerolacton, Carbonaten (z. B. Trimethylencarbonat, Tetramethylencarbonat und dergleichen), Dioxanonen (z. B. 1,4-Dioxanon), 1,Dioxepanonen (z. B. 1,4-Dioxepan-2-on und 1,5-Dioxepan-2-on), Ethylenglykol, Ethylenoxid, Esteramiden, Hydroxyalkanoaten (z. B. γ-Hydroxyvalerat, β-Hydroxypropionat, 3-Hydroxybuterat und dergleichen), Poly(orthoestern), Tyrosincarbonaten, Polyimidcarbonaten, Polyiminocarbonaten wie Poly(bisphenol A-iminocarbonat) und Poly(hydrochinoniminocarbonat), Polyurethanen, Polyanhydriden, Polymerarzneimitteln (z. B. Polydiflunisol, Polyaspirin und Proteintherapeutika) und Copolymeren und Kombinationen davon.

10. Medizinische Vorrichtung nach Anspruch 8, wobei der Kern biologisch abbaubare Polymere umfasst, ausgewählt aus Kollagen, Cellulose, Poly(aminosäuren), Polysacchariden, Hyaluronsäure, Eingeweiden ("gut"), Copolymeren und Kombinationen davon.

11. Medizinische Vorrichtung nach Anspruch 8, wobei er Kern nicht-abbaubare Polymere umfasst, ausgewählt aus fluorierten Polymeren (z. B. Fluorethylenen, Propylenen, fluorPEGs), Polyolefinen wie Polyethylen, Polyestern wie Polyethylenterephthalat (PET), Nylons, Polyamiden, Polyurethanen, Siliconen, Polyethylen mit äußerst hohem Molekulargewicht (UHMWPE), Polybutestern, Polyaryletherketon, Copolymeren und Kombinationen davon.

12. Medizinische Vorrichtung nach Anspruch 8, wobei die funktionelle Gruppe, von der bekannt ist, dass sie Click-Reaktivität besitzt, ein Thiol ist.

13. Medizinische Vorrichtung nach Anspruch 8, wobei die funktionelle Gruppe, von der bekannt ist, dass sie Click-Reaktivität besitzt, ein Azid ist.

14. Medizinische Vorrichtung nach Anspruch 8, wobei die funktionelle Gruppe, von der bekannt ist, dass sie Click-Reaktivität besitzt, ein Alkyn ist.

15. Medizinische Vorrichtung nach Anspruch 8, wobei die funktionelle Gruppe, von der bekannt ist, dass sie Click-Reaktivität besitzt, ein Alken ist.

## Revendications

1. Procédé pour former un dispositif médical comprenant :
la formation d'une forme souhaitée à partir d'un polymère possédant un coeur et au moins un groupe fonctionnel connu pour avoir une réactivité click, de façon qu'un dispositif médical avec une surface activée soit produit, dans lequel le dispositif médical est une fibre et dans lequel le groupe fonctionnel connu pour avoir une réactivité click est choisi dans le groupe constitué par les thiols, les azotures, les alcynes et les alcènes.

2. Procédé selon la revendication 1, dans lequel le coeur comprend des matériaux synthétiques choisis parmi les α-hydroxyacides (par exemple acide lactique, acide glycolique, et analogues), le lactide, le glycolide, l'ε-caprolactone, la δ-valérolactone, les carbonates (par exemple carbonate de triméthylène, carbonate de tétraméthylène, et analogues), les dioxanones (par exemple 1,4-dioxanones), les 1,dioxépanones (par exemple 1,4-dioxépan-2-one et 1,5-dioxépan-2-one), l'éthylèneglycol, l'oxyde d'éthylène, les esteramides, les hydroxyalcanoates (par exemple γ-hydroxyvalérate, β-hydroxypropionate, 3-hydroxybutérate, et analogues), les polyorthoesters, les tyrosinecarbonates, les polyimidocarbonates, les polyiminocarbonates tels que le poly(iminocarbonate de bisphénol A) et le poly(iminocarbonate d'hydroquinone), les polyuréthanes, les polyanhydrides, les médicaments polymères (par exemple polydiflunisol, polyaspirine, et agents thérapeutiques protéiques), ainsi que leurs copolymères et combinaisons.

3. Procédé selon la revendication 1, dans lequel le coeur comprend des polymères biodégradables choisis parmi le collagène, la cellulose, les poly(acides aminés), les polysaccharides, l'acide hyaluronique, les boyaux, ainsi que leurs copolymères et combinaisons.

4. Procédé selon la revendication 1, dans lequel le coeur comprend des polymères non dégradables choisis parmi les polymères fluorés (par exemple fluoroéthylènes, propylènes, PEG fluorés), les polyoléfines telles que le polyéthylène, les polyesters tels que le poly(téréphtalate d'éthylène) (PET), les nylons, les polyamides, les polyuréthanes, les silicones, le polyéthylène de masse moléculaire ultra élevée (UHMWPE), les polybutesters, la polyaryléthercétone, ainsi que leurs copolymères et combinaisons.

5. Procédé selon l'une quelconque des revendications 1 à 4, dans lequel le polymère est extrudé à l'état fondu pour former une fibre.

6. Procédé selon la revendication 1, dans lequel le polymère étant du chitosane, le polymère est formé en une fibre par filage d'une solution anisotrope de chitosane.

7. Procédé selon la revendication 1, dans lequel le polymère étant du collagène, le polymère est formé en une fibre par filage de gel.

8. Dispositif médical comprenant un polymère possédant un coeur et un groupe fonctionnel connu pour avoir une réactivité click, lequel dispositif médical est une fibre, dans lequel le groupe fonctionnel connu pour avoir une réactivité click est choisi dans l'ensemble constitué par les thiols, les azotures, les alcynes et les alcènes.

9. Dispositif médical selon la revendication 8, dans lequel le coeur comprend des matériaux synthétiques choisis parmi les α-hydroxyacides (par exemple acide lactique, acide glycolique, et analogues), le lactide, le glycolide, l'ε-caprolactone, la δ-valérolactone, les carbonates (par exemple carbonate de triméthylène, carbonate de tétraméthylène, et analogues), les dioxanones (par exemple 1,4-dioxanones), les 1,dioxépanones (par exemple 1,4-dioxépan-2-one et 1,5-dioxépan-2-one), l'éthylèneglycol, l'oxyde d'éthylène, les esteramides, les hydroxyalcanoates (par exemple γ-hydroxyvalérate, β-hydroxypropionate, 3-hydroxybutérate, et analogues), les polyorthoesters, les tyrosinecarbonates, les polyimidocarbonates, les polyiminocarbonates tels que le poly(iminocarbonate de bisphénol A), et le poly(iminocarbonate d'hydroquinone), les polyuréthanes, les polyanhydrides, les médicaments polymères (par exemple polydiflunisol, polyaspirine, et agents thérapeutiques protéiques), ainsi que leurs copolymères et combinaisons.

10. Dispositif médical selon la revendication 8, dans lequel le coeur comprend des polymères biodégradables choisis parmi le collagène, la cellulose, les poly(acides aminés), les polysaccharides, l'acide hyaluronique, les boyaux, ainsi que leurs copolymères et combinaisons.

11. Dispositif médical selon la revendication 8, dans lequel le coeur comprend des polymères non dégradables choisis parmi les polymères fluorés (par exemple fluoroéthylènes, propylènes, PEG fluorés), les polyoléfines telles que le polyéthylène, les polyesters tels que le poly(téréphtalate d'éthylène) (PET), les nylons, les polyamides, les polyuréthanes, les silicones, le polyéthylène de masse moléculaire ultra élevée (UHMWPE), les polybutesters, la polyaryléthercétone, ainsi que leurs copolymères et combinaisons.

12. Dispositif médical selon la revendication 8, dans lequel le groupe fonctionnel connu pour avoir une réactivité click est un thiol.

13. Dispositif médical selon la revendication 8, dans lequel le groupe fonctionnel connu pour avoir une réactivité click est un azoture.

14. Dispositif médical selon la revendication 8, dans lequel le groupe fonctionnel connu pour avoir une réactivité click est un alcyne.

15. Dispositif médical selon la revendication 8, dans lequel le groupe fonctionnel connu pour avoir une réactivité click est un alcène.
